# NEUE EUROPÄISCHE PATENTSCHRIFT

(11) **EP 2 233 123 B2**
(45) Veröffentlichungstag und Bekanntmachung des Hinweises auf die Entscheidung über den Einspruch: **11.03.2020**
(45) Hinweis auf die Patenterteilung: 28.11.2012
(21) Anmeldenummer: 09156063.1
(22) Anmeldetag: 24.03.2009
(51) Int. Cl.: A61K 6/00, A61K 6/08

(54) **Selbsthaftender mehrkomponentiger Dentalwerkstoff**
Self-adhesive multi-component dental material
Matière active à plusieurs composants auto-adhésive

(43) Veröffentlichungstag der Anmeldung: 29.09.2010
(73) Patentinhaber: Ivoclar Vivadent AG, 9494 Schaan (LI)
(72) Erfinder: Burtscher, Peter Dr., A-6830, Rankweil (AT); Eder, Marion, 6850, Dornbirn (AT); Kammann, Axel, 6800, Feldkirch (AT)
(74) Vertreter: Uexküll & Stolberg

(56) Entgegenhaltungen:
- EP-A- 0 219 058
- EP-A- 1 066 813
- EP-A1- 0 554 890
- EP-A1- 1 269 968
- EP-A1- 1 393 705
- EP-A1- 1 479 364
- EP-A1- 1 502 569
- EP-A1- 1 780 223
- EP-A1- 1 878 418
- EP-A1- 2 108 663
- EP-A1- 2 233 123
- EP-A2- 0 219 058
- EP-A2- 0 408 357
- WO-A1-02/092021
- WO-A1-2008/087977
- DE-A1- 19 906 834
- DE-A1- 19 906 834
- JP-A- 2003 013 012
- JP-A- 2008 189 579
- US-A1- 2007 203 257
- US-A1- 2008 015 279
- US-B1- 6 472 454
- US-B2- 6 732 887
- US-B2- 6 984 673
- US-B2- 7 156 911
- "RelyX Unicem Clicker, RelyX Unicem Aplicap", 3M, 2007,
- "RelyX Unicem, Self-Adhesive Universal Resin Cement", 3M, 2002,
- "RelyX Unicem, Aplicap/Maxicap, Selbstadhäsiver universaler Befestigungszement", 3M, December 2002 (2002-12),
- Stawarczyck et al: Journal of Prosthetic Dentistry, vol. 107, no. 2, 2012, pages 94-101,
- Quarzwerke, Silmikron VP 805-10/1, 2 Seiten (08/03) & Quarzwerke, Sikron-Feinstmehle Cristobalit, 2 Seiten (10/02) & Silbond 904-002, 2 Seiten (01/01) & EG-Sicherheitsdatenblatt Cristobalitmehl, 5 Seiten, 24052004 & EG-Sicherheitsdatenblatt Oberflächenmodifiziertes Cristobalitmehl, 5 Seiten, 24052004 & Sicherheitsdatenblatt Sipernat 880, 07052002 & Sicherheitsdatenblatt Sipernat D10, 04.11.2002
- Versuchsbericht von Herrn A. Gianasmidis (Nacharbeitung von Beispiel 5 der D6), 19032015, 3 Seiten
- Schott DentalGlass/ Inert, Datenblatt, 2 Seiten
- Messung der Haftung von Tetric EvoCeram an Rinderdentin, 2 Seiten
- Burtscher, P.: Untersuchungsbericht der ISO/TC 106, 7 February 2014 (2014-02-07), pages 1-4,
- Burtscher, P.: "Abstract 2794", IADR General Session and Exhibition, Deattle 20-23.03.2013,
- RelyX Unichem 2 Automix, Produktbroschüre 3M ESPE, 8 Seiten, 2010

## Beschreibung

Die vorliegende Erfindung betrifft einen selbsthaftenden Dentalwerkstoff auf Paste/Paste-Basis, vorzugsweise ein Befestigungskomposit.

In der Zahnheilkunde werden für die Befestigung von dentalen Restaurationen, wie Kronen, Brücken, Inlays, auf der Zahnhartsubstanz häufig Befestigungsmaterialien auf Kompositbasis verwendet. Unter dentalen Kompositen versteht man allgemein Mehrstoffsysteme, die aus einer organischen Monomer- bzw. Polymermatrix bestehen, in der ein oder mehrere Füllstoffe eingebaut sind. Für einen ausreichenden Verbund zur Zahnhartsubstanz werden die Befestigungskomposite in Kombination mit einem sauren Dentinhaftvermittler eingesetzt, d.h. vor dem Aufbringen des Komposits wird die Zahnhartsubstanz mit dem sauren Dentinhaftvermittler behandelt.

Eine Vereinfachung des Behandlungsablaufs wird erreicht, wenn auf den Dentinhaftvermittler verzichtet wird und das Befestigungskomposit alleine zur Anwendung gelangen kann. Diese sogenannten selbsthaftenden oder selbstadhäsiven Befestigungskomposite enthalten in der Monomermatrix zusätzlich saure Monomere, welche eine Haftung zur Zahnhartsubstanz ermöglichen. Häufig handelt es sich um dualhärtende Systeme, die neben einem Photoinitiator zur lichtinduzierten Härtung ein Initiatorsystem zur Selbsthärtung bei Raumtemperatur aufweisen. Die Selbsthärtung des Befestigungskomposits ist vor allem dann notwendig, wenn die zu befestigende Restauration (z.B. Krone, Brücke) sehr opak (z.B. Zirkonoxid) oder sogar lichtundurchlässig (z.B. Metall) ist.

In der Regel sind die selbsthaftenden Befestigungskomposite Systeme auf Pulver/Flüssigkeits-Basis, welche sehr lagerstabil sind, da Teile des Initiatorsystems auf das Pulver beschichtet werden können. Da Pulver/Flüssigkeits-Systeme in der Anwendung aufwendig sind, ein Mischgerät zur Anmischung erfordern, der Mischvorgang bei hohen Drehzahlen häufig zu Lufteinschlüssen führt und sie hinsichtlich der Füllmenge keine große Variabilität aufweisen, gehören diese nicht zu den bevorzugten Lieferformen.

Eine Paste/Paste-Darreichung hat den Vorteil, dass keine speziellen Zusatzgeräte erforderlich sind und jede beliebige Menge an Befestigungsmaterial problemlos angemischt werden kann. In jüngerer Zeit sind einige Materialien auf dieser Basis auf den Markt gekommen, aber allen ist gemeinsam, dass die Dentinhaftungseigenschaften dieser kommerziell erhältlichen Systeme bei weitem nicht an jene von Pulver/Flüssigkeits-Systemen heranreichen, und die meisten von diesen Paste/Paste-Darreichungen weisen nach Selbsthärtung ohne anschließende Lichthärtung fast keine Dentinhaftung auf. Die bekannten Befestigungskomposite auf Paste/Paste-Basis zeigen ferner häufig Probleme in der Lagerstabilität, das heißt die Dentinhaftungseigenschaften sind nach Lagerung der Pasten über einen längeren Zeitraum nicht mehr gewährleistet.

EP 1 479 364 A1 und EP 1 502 569 A1 beschreiben selbsthaftende Paste/Paste-Dentalwerkstoffe, die selbsthärtend oder dualhärtend sind. In diesen Anmeldungen wurde versucht, die Lagerstabilität durch die Verwendung spezieller Initiatorsysteme und deren Aufteilung auf die beiden Pasten zu verbessern. In EP 1 479 364 A1 kommt eine Kombination eines Hydroperoxids mit einem bestimmten substituierten Thioharnstoff zum Einsatz, und in EP 1 502 569 A1 werden Kombinationen von Oxidationsmitteln wie Hydroperoxiden, Cu(II)-, Fe(III)-, Co(III)-Salzen, Persulfaten und Permanganaten mit Reduktionsmitteln wie z.B. Sulfinaten, Thioharnstoff, substituierten Thioharnstoffen, Ascorbinsäure und deren Derivaten, Barbitursäure und deren Derivaten, Thiobarbitursäure und deren Derivaten, Fe(II)-, Cu(I)-und Co(II)-Salzen offenbart. Es wird in beiden Anmeldungen allgemein eine Vielzahl unterschiedlicher Füllstoffe beschrieben, ohne dass bevorzugte Füllstoffe hervorgehoben werden, und auch die Zuordnung der Füllstoffe zu der jeweiligen Paste des Zweikomponentensystems ist nicht Gegenstand der Lehre. Die in EP 1 479 364 A1 und EP 1 502 569 A1 beschriebenen Dentinhaftwerte nach Aushärtung lassen sich in der Praxis nicht erreichen. Insbesondere nach Lagerung können nur Dentinhaftwerte von 0 bis 2 MPa erzielt werden.

Die DE 199 06 834 A1 offenbart dentale Glasionomerzemente vom Pastentyp, die eine erste und eine zweite Paste umfassen. Die erste Paste enthält ein α,β-ungesättigtes Carbonsäurepolymer, Wasser und Füllstoff, der nicht mit dem Carbonsäurepolymer reagiert. Die zweite Paste enthält Fluoraluminosilikatglaspulver und säuregruppenfreies, polymerisierbares Monomer. Das Carbonsäurepolymer hat ein Molekulargewicht von 5.000 bis 40.000.

Die EP 0 219 058 A2 offenbart polymerisierbare Zementmischungen, die polymerisierbare, ungesättigte, säuregruppenhaltige Monomere, Oligomere oder Prepolymere, reaktive Füllstoffe und Härtungsmittel enthalten. Die Zemente härten sowohl radikalisch als auch durch Ionenreaktionen aus.

Aufgabe der vorliegenden Erfindung ist es, einen selbsthaftenden Dentalwerkstoff auf Paste/Paste-Basis bereitzustellen, der gegenüber den bekannten Paste/Paste-Systemen verbesserte Dentinhafteigenschaften, insbesondere auch nach Lagerung, zeigt.

Die Aufgabe wird erfindungsgemäß gelöst durch einen härtbaren mehrkomponentigen Dentalwerkstoff, umfassend
(A) eine erste Paste, enthaltend
   (i) ein oder mehrere säuregruppenfreie radikalisch polymerisierbare Monomere und
   (ii) basischen Füllstoff,
   (vii) basischen Aktivator,
   (viii) Photoinitiator und
   (ix) nicht basischen Füllstoff,
(B) eine zweite Paste, enthaltend
   (iii) ein oder mehrere säuregruppenhaltige radikalisch polymerisierbare Monomere,
   (iv) nichtbasischen Füllstoff,
   (v) Peroxid und
   (vi) ein oder mehrere säuregruppenfreie radikalsich polymerisierbare Monomere,
wobei Paste (A) keine säuregruppenhaltigen radikalisch polymerisierbare Monomere und Paste (B) keinen basischen Füllstoff enthält und
wobei der Dentalwerkstoff kein zugesetztes Lösungsmittel enthält.

Die Erfindung betrifft auch ein Verfahren zur Bereitstellung eines gebrauchsfertigen Dentalwerkstoffs durch Vermischen der Paste (A) und der Paste (B) des oben beschriebenen härtbaren mehrkomponentigen Dentalwerkstoffs.

Gegenstand der vorliegenden Erfindung ist ferner ein oben beschriebener härtbarer mehrkomponentiger Dentalwerkstoff zur Verwendung in der Zahnheilkunde, insbesondere zur Befestigung einer dentalen Restauration an Zahnschmelz und/oder Dentin. Es wird ein Verfahren beschrieben, bei dem man Paste (A) und Paste (B) des oben beschriebenen härtbaren mehrkomponentigen Dentalwerkstoffs vermischt und man die erhaltene Mischung dann auf die Zahnhartsubstanz, d.h. Zahnschmelz und/oder Dentin aufbringt.

Paste (A) enthält keine säuregruppenhaltigen radikalisch polymerisierbaren Monomere und Paste (B) keinen basischen Füllstoff.

Die Paste (B) enthält neben den säuregruppenhaltigen radikalisch polymerisierbaren Monomeren (iii) zusätzlich ein oder mehrere säuregruppenfreie radikalisch polymerisierbare Monomere (vi). Dabei können die säuregruppenfreien radikalisch polymerisierbaren Monomere in Paste (B) gleich oder unterschiedlich zu denen in Paste (A) sein. Die Paste (A) enthält als Füllstoff eine Mischung von basischem Füllstoff und nichtbasischem Füllstoff. Dabei kann der nichtbasische Füllstoff in Paste (A) gleich oder unterschiedlich zu dem in Paste (B) sein.

Der erfindungsgemäße mehrkomponentige Dentalwerkstoff ist dualhärtend, d.h. selbsthärtend und photohärtbar.

Unter einem "basischen Füllstoff" im Sinne der Erfindung versteht man einen Füllstoff, der, wenn er in einer Menge von 2 g mit einer Teilchengröße, wie sie in dem erfindungsgemäßen Dentalwerkstoff eingesetzt werden soll, in 50 ml destilliertem Wasser (pH = 7) durch Rühren bei ca. 20 °C suspendiert wird, nach 30 min eine Erhöhung des pH-Werts des Wassers auf mindestens 8, bevorzugt mindestens 8,5 bewirkt. Umgekehrt versteht man hierin unter einem "nichtbasischen Füllstoff" einen Füllstoff, der, wenn er in einer Menge von 2 g mit einer Teilchengröße, wie sie in dem erfindungsgemäßen Dentalwerkstoff eingesetzt werden soll, in 50 ml destilliertem Wasser (pH = 7) durch Rühren bei ca. 20 °C suspendiert wird, nach 30 min eine Erhöhung des pH-Werts des Wassers auf weniger als 8, bevorzugt weniger als 7,5 und besonders bevorzugt keine Erhöhung des pH-Werts bewirkt, wobei eine Erniedrigung des pH-Werts erlaubt ist.

Typische nichtbasische Füllstoffe, die zum Einsatz in Paste (B) bzw. optional in Paste (A) des erfindungsgemäßen Dentalwerkstoffs geeignet sind, sind Siliciumdioxid in verschiedenen Modifikationen und neutrale Metallsalze. Bevorzugt sind unter den SiO₂-Modifikationen, pyrogene Kieselsäure, Fällungskieselsäure, Sol-Gel-Silica, Quarz (insbesondere natürlicher Quarz) und Quarzglas. Alle SiO₂-Modifikationen können oberflächenbehandelt sein. Unter Oberflächenbehandlung sind sowohl übliche Hydrophobierungen mit entsprechenden Siliconen, Silanen und/oder Silazanen, z.B. Hexamethyldisilazan, als auch Funktionalisierungen der Oberfläche mit polymerisationsfähigen Silanen wie unten bei den alkalischen Gläsern beschrieben zu verstehen. Beispiele für neutrale Metallsalze umfassen neutrale Fluoride der Seltenerdmetalle, vorzugsweise Ytterbiumtrifluorid, aber auch andere Metallsalze, wie etwa Bariumsulfat, Strontiumfluorid, Bariumfluorid, Yttriumfluorid und Bariumwolframat.

Weitere Beispiele für nichtbasische Füllstoffe sind Mischoxide von SiO₂ mit ZrO₂, Ta₂O₃ und/oder Ta₂O₅.

Geeignete nichtbasische Füllstoffe sind ferner sogenannte "Isofüller", d.h. Pulver, die durch Mahlen von gehärteten Kompositen erhalten werden, die nichtbasischen Füllstoff enthalten, der vorzugsweise aus den oben genannten nichtbasischen Füllstoffen ausgewählt ist. Die Polymermatrix des Isofüllers kann aus anderen oder aus den gleichen Monomeren hergestellt werden, die in dem System vorhanden sind, in dem er eingesetzt wird, allerdings ohne säuregruppenhaltige Monomere. Es ist nicht notwendig im dem Isofüller den gleichen Füllstoff, bzw. Füllstoff(e) der gleichen Art zu verwenden wie in dem System vorhanden, in dem der Isofüller eingesetzt wird; aus optischen Gründen kann dies allerdings Vorteile haben.

Der nichtbasische Füllstoff kann aus einer einzigen Art von nichtbasischem Füllstoff oder auch aus Kombinationen von verschiedenen Arten von nichtbasischen Füllstoffen, vorzugsweise den oben genannten, bestehen. Besonders bevorzugt sind Ytterbiumtrifluorid, Fällungskieselsäure, Sol-Gel-Silica, Isofüller, pyrogene Kieselsäure und deren Kombinationen. Beispiele für geeignete Füllerkombinationen sind (a) Ytterbiumtrifluorid, Isofüller und pyrogene Kieselsäure und (b) Fällungskieselsäure (oder Sol-Gel-Silica), Ytterbiumtrifluorid und pyrogene Kieselsäure.

Bevorzugte basische Füllstoffe, die zum Einsatz in Paste (A) des erfindungsgemäßen Dentalwerkstoffs geeignet sind, sind alkalische Gläser, etwa Silicatgläser, wie z.B. Aluminosilicat-Gläser, Aluminoborosilicat-Gläser, Aluminium-Fluoro-Silicat-Gläser, insbesondere Gläser, die Elemente der 1. und 2. Hauptgruppe des Periodensystems der Elemente enthalten, wie Barium-Silicat-Gläser, Barium-Aluminium-Silicat-Gläser (z.B. der Zusammensetzung 55 Gew.-% SiO₂, 25 Gew.-% BaO, 10 Gew.-% B₂O₃ und 10 Gew.-% Al₂O₃), Strontium-Silicat-Gläser, Strontium-Aluminium-Silicat-Gläser und Lithium-Aluminium-Silicat-Gläser, einschließlich jeweils der entsprechenden fluorhaltigen Gläser.

Die alkalischen Gläser können optional silanisiert sein. Unter "Silanisierung" versteht man die Funktionalisierung der Glasoberfläche mit polymerisationsfähigen Silanen, etwa durch Umsetzung mit (meth)acrylatfunktionalisierten Silanen, z.B. (Meth)acryloyloxyalkyltrialkoxysilanen, üblicherweise 3-(Methacryloyloxy)propyltrimethoxysilan, 3-(Methacryloyloxy)propyltriethoxysilan, 3-(Methacryloyloxy)propyltrichlorsilan, Methacryloyloxymethyltrimethoxysilan, Methacryloyloxymethyltriethoxysilan, 3-(Methacryloyloxy)propylmethyldichlorsilan oder 3-(Methacryloyloxy)propylmethyldimethoxysilan. 3-(Methacryloyloxy)propyltrimethoxysilan ist bevorzugt.

Auch Isofüller wie zuvor beschrieben, die in diesem Fall aber basischen Füllstoff, vorzugsweise aus den oben genannten genannten, enthalten, sind als basischer Füllstoff (ii) im Sinne der Erfindung geeignet.

Der basische Füllstoff kann aus einer einzigen Art von basischem Füllstoff oder auch aus Kombinationen von verschiedenen Arten von basischen Füllstoffen, vorzugsweise den oben genannten, bestehen.

Die oben genannten nichtbasischen und basischen Füllstoffe auf Basis von Quarz, Quarzglas, alkalischen Gläsern und Glaskeramiken sind Pulver mit einer bevorzugten gewichtsmittleren Partikelgröße im Bereich von 0,01 bis 10 µm, besonders bevorzugt von 0,2 bis 5 µm und ganz besonders bevorzugt von 0,6 bis 2,0 µm. Die obengenannten nichtbasischen und basischen Füllstoffe auf Basis von pyrogener Kieselsäure, Fällungskieselsäure, Sol-Gel-Silica und Seltenerdfluoriden sind nanopartikuläre oder mikrofeine Pulver mit einer bevorzugten gewichtsmittleren Partikelgröße im Bereich von 10 nm bis 5 µm, besonders bevorzugt von 20 nm bis 5 µm und ganz besonders bevorzugt 40 bis 300 nm. Die obengenannten Isofüller sind Pulver mit einer bevorzugten gewichtsmittleren Partikelgröße im Bereich von 3 bis 100 µm, besonders bevorzugt von 3 bis 20 µm und ganz besonders bevorzugt 3 bis 10 µm. Die obengenannten Füllstoffe aus SiO₂-Mischoxiden sind Pulver mit einer bevorzugten gewichtsmittleren Partikelgröße im Bereich von 100 bis 300 nm.

Das (die) säuregruppenfreie(n) radikalisch polymerisierbare(n) Monomer(e) (i) und (vi) und das (die) säuregruppenhaltige(n) radikalisch polymerisierbare(n) Monomer(e) (iii) machen gemeinsam das Bindemittel des erfindungsgemäßen Dentalwerkstoffs aus, das beim Aushärten radikalisch polymerisiert.

Die säuregruppenfreien radikalisch polymerisierbaren Monomere (i) und (vi) sind vorzugsweise sogenannte Vernetzermonomere mit mindestens zwei, vorzugsweise 2 bis 4, radikalisch polymerisierbaren Gruppen, bevorzugt Acryloyl- und Methyacryloylgruppen. Geeignete Beispiele umfassen 1,6-Bis[2-methacryloyloxyethoxycarbonylamino]-2,4,4-trimethylhexan (UDMA), Diethylenglykoldimethacrylat, Triethylenglykoldimethacrylat (TEGDMA), Polyethylenglykoldimethacrylat, z.B. PEG-400-Dimethacrylat, 2,2-Bis[4-(2-hydroxy-3-methacryloyloxypropoxy)phenyl]propan (Bis-GMA), 1,6-Bis[2-acryloyloxyethoxycarbonylamino]-2,4,4-trimethylhexan, Diethylenglykoldiacrylat, Triethylenglykoldiacrylat, Polyethylenglykoldiacrylat, z.B. PEG-400-Diacrylat, Trimethylolpropantrimethacrylat, Trimethylolpropantriacrylat, Pentaerythrittetramethacrylat, Pentaerythrittetraacrylat, propoxyliertes Bisphenol-A-Dimethacrylat sowie Butandioldimethacrylat, Butandioldiacrylat, 1,10-Decandioldimethacrylat (D₃MA), 1,10-Decandioldiacrylat, 1,12-Dodecandioldimethacrylat und 1,12-Dodecandioldiacrylat, die durch Veresterung von (Meth)acrylsäure mit den entsprechenden Di- oder Polyolen zugänglich sind. Darüber hinaus sind auch hydrolysestabile Vernetzermonomere geeignet, beispielsweise Urethane aus 2-(Hydroxymethyl)acrylsäure und Diisocyanten, wie 2,2,4-Trimethylhexamethylendiisocyanat und Isophorondiisocyanat; vernetzende Pyrrolidone, wie z.B. 1,6-Bis(3-vinyl-2-pyrrolidonyl)-hexan; kommerziell zugängliche Bisacrylamide bzw. Bis(meth)acrylamide, wie Methylen- und Ethylenbisacrylamid, N,N'-Diethyl-1,3-bis(acrylamido)-propan, 1,3-Bis-(methacrylamido)-propan, 1,4-Bis(acrylamido)-butan und 1,4-Bis(acryloyl)-piperazin, die durch Umsetzung aus den entsprechenden Diaminen mit (Meth)acrylsäurechlorid synthetisiert werden können.

Als "hydrolysestabil" werden hierin solche Monomere bezeichnet, die in Wasser oder in Mischungen von Wasser und wassermischbaren Lösungsmitteln bei einer Konzentration von ca. 20 Gew.-% und einem pH-Wert von ca. 2,0 bei 37°C für mindestens 6 Wochen stabil sind, d.h. zu weniger als 5 % hydrolysieren.

Bevorzugt werden Mischungen verschiedener Vernetzermonomere eingesetzt, z.B. eine Mischung aus TEGDMA, PEG-400-Dimethacrylat und UDMA, und optional noch propoxyliertem Bisphenol-A-Dimethacrylat.

Zusätzlich zu einem oder mehreren Vernetzermonomeren können als säuregruppenfreie radikalisch polymerisierbare Monomere (i) oder (vi) noch eine oder mehrere sogenannte Verdünnermonomere vorhanden sein, die sich aufgrund ihrer Viskosität und guten Löslichkeit zum Verdünnen von Polymerisationsharzen eignen. Verdünnermonomere können eine oder auch mehrere polymerisierbare Gruppen enthalten und im letzteren Fall auch als Vernetzermonomere wirken.

Als Verdünnermonomere werden hierin bevorzugt flüssige Monomere mit einer Viskosität η von kleiner als 100 mPa·s, gemessen bei 20°C, verwendet. Beispiele für Verdünnermonomere umfassen hydrolysestabile Mono(meth)acrylate, z.B. Mesitylmethacrylat; 2-(Alkoxymethyl)acrylsäuren, z.B. 2-(Ethoxymethyl)acrylsäure, 2-(Hydroxymethyl)acrylsäure; N-mono- oder -disubstitiuierte Acrylamide, wie z.B. N-Ethylacrylamid, N,N-Dimethylacrylamid, N-(2-Hydroxyethyl)acrylamid und N-Methyl-N-(2-hydroxyethyl)-acrylamid bzw. N-monosubstituierte Methacrylamide, wie z.B. N-Ethylmethacrylamid und N-(2-Hydroxyethyl)methacrylamid. Außerdem lassen sich auch N-Vinylpyrrolidon, 2-(Methacryloyloxy)-ethylacetoacetat oder Allylether als Verdünnermonomere einsetzen. In vielen Fällen jedoch ist die Anwesenheit eines Verdünnermonomers weder in Paste (A) noch in Paste (B) des erfindungsgemäße Dentalwerkstoffs notwendig.

Das (die) säuregruppenhaltige(n) radikalisch polymerisierbare(n) Monomer(e) (iii) ist (sind) für eine verbesserte Haftung des Dentalwerkstoffs an Schmelz/Dentin verantwortlich. Es handelt sich hierbei um stark saure Monomere, auch Adhäsivmonomere genannt, die zum einen bei der Anwendung des Dentalwerkstoffs eine leichte Schmierschicht ("Smear-Layer") auf dem Schmelz/Dentin entfernen und zum anderen den Schmelz bzw. das Dentin anätzen, so dass Monomere eindiffundieren können und bei der anschließenden Polymerisation unter Bildung von sogenannten Polymer-Tags zu einer starken Bindung zwischen dem gehärteten Dentalwerkstoff und Schmelz/Dentin führen.

Die säuregruppenhaltigen radikalisch polymerisierbaren Monomere (iii) enthalten mindestens eine ethylenisch ungesättigte Gruppe, vorzugsweise ausgewählt aus Acryloyl-, Methacryloyl-, Vinylgruppen und deren Kombinationen, und mindestens eine, vorzugsweise 1 bis 4 Säuregruppen. Bevorzugte Säuregruppen sind Carbonsäure-, Sulfonsäure-, Phosphonsäure- und/oder Phosphorsäuregruppen, wobei die jeweils teilveresterten Säuregruppen, die noch acide Wasserstoffatome aufweisen mitumfasst sind. Verbindungen, die als Säuregruppen Phosphonsäure- und/oder Phosphorsäuregruppen enthalten, sind besonders bevorzugt. Verbindungen mit mehr als einer Säuregruppen können unterschiedliche Säuregruppen oder vorzugsweise identische Säuregruppen enthalten. Besonders vorteilhafte säuregruppenhaltige radikalisch polymerisierbare Monomere (iii) sind polymerisationsfähige Dihydrogen- und Hydrogenphosphate.

Geeignete Beispiele für säuregruppenhaltige radikalisch polymerisierbare Monomere (iii) umfassen Glycerin-dimethacrylatdihydrogenphosphat (GDMP), 4-(Meth)acryloyloxyethyl-trimellitsäureanhydrid, 10-Methacryloyloxydecylmalonsäure, N-(2-Hydroxy-3-meth-acryloyloxypropyl)-N-phenylglycin, 4-Vinylbenzoesäure, 2-Methacryloyloxyethyl-phenyl-hydrogenphosphat, 10-Methacryloyloxydecyl-dihydrogenphosphat (MDP), 2-Methacryloyloxyethyl-dihydrogenphosphat (HEMA-Phosphat), Di(2-methacryloyloxyethyl)-hydrogenphosphat (Di-HEMA-Phosphat), (2-Methacryloyloxypropyl-dihydrogenphosphat, Dipentaerythritpentamethacryloyloxyphosphat und Phosphorsäuremono-(1-acryloyl-piperidin-4-yl)-ester. Vorteilhaft sind auch hydrolysestabile Adhäsivmonomere, wie 4-Vinylbenzylphosphonsäure, 2-[4-(Dihydroxyphosphoryl)-2-oxa-butyl]-acrylsäure sowie deren Amide und hydrolysestabile Ester, wie z.B. 2-[4-(Dihydroxy-phosphoryl)-2-oxa-butyl]-acrylsäure-2,4,6-trimethylphenyl-ester, sowie (Meth)acrylamiddihydrogenphosphate, wie z.B. 6-Methacrylamidohexyl- und 1,3-Bis(methacrylamido)-propan-2-yl-dihydrogen-phosphat und 1,3-Bis-(N-acryloyl-N-propyl-amino)-propan-2-yl-dihydrogenphosphat. Weitere Beispiele umfassen Phosphonsäuremonomere wie etwa Vinylphosphonsäure, 4-Vinylphenylphosphonsäure, 2-Methacryloyloxyethylphosphonsäure, 2-Methacrylamidoethylphosphonsäure, 4-Methacrylamido-4-methylpentyl-phosphonsäure, 2-[2-Dihydroxyphosphoryl)-ethoxymethyl]-acrylsäure-ethyl- und -2,4,6-trimethylphenylester. Darüber hinaus eignen sich polymerisationsfähige Sulfonsäuren als Haftmonomere, insbesondere Vinylsulfonsäure, 4-Vinylphenylsulfonsäure oder 3-(Methacrylamido)propylsulfonsäure.

Der erfindungsgemäße Dentalwerkstoff ist dualhärtend, d.h. selbsthärtend und photochemisch härtbar. Unter Strahlungshärtung wird vorzugsweise Härtung durch sichtbares Licht, insbesondere Blaulicht mit einer Wellenlänge von 300 bis 550 nm, vorzugsweise 400 bis 500 nm verstanden.

Für die Selbsthärtung werden Initiatoren mit einem entsprechenden Aktivator (vii) kombiniert. Als Initiator (v) für die Selbsthärtung wird ein Peroxid, insbesondere Benzoylperoxid eingesetzt. Als Aktivator (vii) dient in diesem Fall ein basischer Aktivator, vorzugsweise ein reduzierendes Amin, besonders bevorzugt ein aromatisches Amin, wie etwa N,N-Dimethyl-sym.-xylidin oder N,N-Dimethyl-p-toluidin. Es versteht sich von selbst, dass der Initiator (v) und der Aktivator (vii) in unterschiedlichen Pasten vorhanden sein müssen, um ein unerwünschte vorzeitige Polymerisation zu verhindern. Da eine Initiator/Aktivator-Kombination mit einem basischen Aktivator, wie etwa Benzoylperoxid/aromatisches Amin, verwendet wird, ist der Aktivator (vii) in Paste (A) und der Initiator (v) in Paste (B) eingebracht.

Beispiele für geeignete Photoinitiatoren (viii) umfassen Benzophenon, Benzoin sowie deren Derivate und α-Diketone und deren Derivate, wie 9,10-Phenanthrenchinon, 1-Phenyl-propan-1,2-dion, Diacetyl und 4,4'-Dichlorbenzil. Ferner sind Acylposphinoxide, wie etwa 2,4,6-trimethylbenzoyldiphenylphosphinoxid, und Bisacylphosphinoxide, wie etwa Bis(2,4,6-trimethylbenzoyl)phenylphosphinoxid, geeignet. Bevorzugt werden Campherchinon, 2,2-Methoxy-2-phenyl-acetophenon oder α-Diketone jeweils in Kombination mit einem Aktivator in Form eines reduzierenden Amins, wie z.B. 4-(Dimethylamino)-benzoesäureester, N,N-Dimethylaminoethylmethacrylat, N,N-Dimethyl-sym.-xylidin oder Triethanolamin. Weitere geeignete Photoinitiatoren sind Monobenzoyl- oder Dibenzoylgermanium-Derivate.

Für die duale Härtung wird eine Initiator/Aktivator-Kombination für die Selbsthärtung mit einem Photoinitiator kombiniert. In diesem Fall kann auch ein gemeinsamer Aktivator für sowohl den Initiator für die Selbsthärtung als auch für den Photoinitiator eingesetzt werden.

Der Photoinitiator (viii) ist in Paste (A) vorhanden. Weil ein basischer Aktivator für den Photoinitiator (viii), z.B. ein Amin, verwendet wird, wird der Aktivator (vii) in Paste (A) eingebracht. Da bei einem durch Licht aktivierten Initiatorsystem keine Notwendigkeit besteht, Initiator und Aktivator zu trennen, können Initiator und Aktivator in derselben Paste eingebracht sein. Der erfindungsgemäße Dentalwerkstoff enthält kein zugesetztes Lösungsmittel.

Der mehrkomponentige Dentalwerkstoff der vorliegenden Erfindung ist typischerweise ein 2-Komponenten-Dentalwerkstoff. Es handelt sich also vorzugsweise um einen 2-Komponenten-Dentalwerkstoff auf Paste/Paste-Basis.

Unter Paste versteht man hierin ein Feststoff-FlüssigkeitsGemisch (Suspension) mit einem hohen Gehalt an Feststoffen. Pasten sind nicht mehr fließfähig, sondern streichfest. Sie haben eine teigige Konsistenz.

Paste (A) des erfindungsgemäßen Dentalwerkstoffs weist vorzugsweise einen Füllstoffgehalt von 40 bis 90 Gew.-%, besonders bevorzugt von 60 bis 85 Gew.-%, ganz besonders bevorzugt von 65 bis 80 Gew.-% und am meisten bevorzugt von 70 bis 80 Gew.-% auf, jeweils bezogen auf die Gesamtmasse der Paste (A). Der Gewichtsanteil des nichtbasischen Füllstoffs am Gesamtfüllstoff beträgt dabei vorzugsweise weniger als 50 %, bevorzugt weniger als 30 %.

Paste (B) des erfindungsgemäßen Dentalwerkstoffs weist vorzugsweise einen Füllstoffgehalt von 40 bis 90 Gew.-%, besonders bevorzugt von 60 bis 85 Gew.-% und ganz besonders bevorzugt von 60 bis 70 Gew.-% auf, jeweils bezogen auf die Gesamtmasse der Paste (B).

Der Teil der Pasten (A) und (B), der nicht Füllstoff ist, wird im Folgenden als Matrixmischung bezeichnet. Die Matrixmischungen der Pasten (A) und (B) bestehen im Wesentlichen aus den radikalisch polymerisierbaren Monomeren und gegebenenfalls Initiator(en), Aktivator(en) und weiteren optionalen Bestandteilen.

Die Matrixmischung der Paste (A) enthält säuregruppenfreie radikalisch polymerisierbare Monomere (i) in einer Gesamtmenge von vorzugsweise 88 bis 100 Gew.-%, insbesondere 88 bis 99,93 Gew.-%, besonders bevorzugt von 94 bis 99,85 Gew.-% und ganz besonders bevorzugt von 94,9 bis 99,7 Gew.-%, jeweils bezogen auf die Masse der Matrixmischung der Paste (A). Vorzugsweise enthält die Matrixmischung der Paste (A) Aktivator in einer Gesamtmenge von 0,05 bis 10 Gew.-%, bevorzugt von 0,1 bis 5,0 Gew.-% und besonders bevorzugt von 0,2 bis 4,5 Gew.-%, jeweils bezogen auf die Masse der Matrixmischung der Paste (A). Vorzugsweise enthält die Matrixmischung der Paste (A) Photoinitiator in einer Menge von 0,02 bis 2,0 Gew.-%, bevorzugt von 0,05 bis 1,0 Gew.-% und besonders bevorzugt von 0,1 bis 0,6 Gew.-%, jeweils bezogen auf die Masse der Matrixmischung der Paste (A).

Vorzugsweise enthält Paste (B) säuregruppenhaltige radikalisch polymerisierbare Monomere (i) in einer Gesamtmenge von 3 bis 40 Gew.-%, besonders bevorzugt von 4 bis 30 Gew.-% und ganz besonders bevorzugt von 5 bis 15 Gew.-%, jeweils bezogen auf die Masse der Matrixmischung der Paste (B). Die Matrixmischung der Paste (B) enthält die säuregruppenfreien radikalisch polymerisierbaren Monomere (vi) vorzugsweise in einer Gesamtmenge von 60 bis 96,7 Gew.-%, besonders bevorzugt von 70 bis 95,95 Gew.-% und ganz besonders bevorzugt von 85 bis 94,9 Gew.-%. Vorzugsweise enthält die Matrixmischung der Paste (B) Initiator (v) für die Selbsthärtung in einer Menge von 0,3 bis 5 Gew.-%, bevorzugt von 0,7 bis 3 Gew.-% und besonders bevorzugt von 1,0 bis 2,0 Gew.-%, jeweils bezogen auf die Masse der Matrixmischung der Paste (B). Optional kann die Matrixmischung der Paste (B) zusätzlich oder alternativ zu dem Initiator für die Selbsthärtung Photoinitiator (viii) in einer Menge von 0,02 bis 2,0 Gew.-%, bevorzugt von 0,05 bis 1,0 Gew.-% und besonders bevorzugt von 0,1 bis 0,6 Gew.-% enthalten, jeweils bezogen auf die Masse der Matrixmischung der Paste (B).

Eine bevorzugten Ausführungsform betrifft einen Dentalwerkstoff bei dem die Paste (A):
65 bis 80 Gew.-%, besonders bevorzugt 70 bis 80 Gew.-%, einer Kombination von basischem Füllstoff (ii) und nichtbasischem Füllstoff (ix) und
20 bis 35 Gew.-%, besonders bevorzugt 20 bis 30 Gew.-%, der oben definierten Matrixmischung für Paste (A) enthält, jeweils bezogen aus die Gesamtmasse der Paste (A), und
die Paste (B):
60 bis 70 Gew.-% nichtbasischen Füllstoff (iv),
30 bis 40 Gew.-% der oben definierten Matrixmischung für Paste (B) enthält,
jeweils bezogen aus die Gesamtmasse der Paste (B).

Aus dem mehrkomponentigen Dentalwerkstoff der vorliegenden Erfindung kann durch einfaches Vermischen der Pasten (A) und (B) ein gebrauchsfertiger Dentalwerkstoff hergestellt werden. Das Vermischen kann z.B. von Hand, etwa mit einem Spatel auf einem Mischblock, erfolgen. Alternativ können die beiden Pasten (A) und (B) auch in einer Doppelspritze vorgelegt werden, um dann bei Gebrauch mit einer Automischkanüle automatisch vermischt zu werden. Vorzugsweise erfolgt das Vermischen der Pasten (A) und (B) im einem Volumenverhältnis im Bereich von 0,4:0,6 bis 0,6:0,4, besonders bevorzugt im Bereich von 0,43:0,57 bis 0,57:0,43, ganz besonders bevorzugt im Bereich von 0,46:0,54 bis 0,54:0,46 und am meisten bevorzugt in einem Volumenverhältnis von etwa 1:1. Der erfindungsgemäße Dentalwerkstoff kann als Befestigungskomposit, z.B. für die Befestigung von Kronen, Brücken, Wurzelstiften und Inlays verwendet werden. Der erfindungsgemäße Dentalwerkstoff ist selbsthaftend, d.h. er kann ohne die Verwendung eines Haftvermittlers direkt auf die Zahnhartsubstanz aufgebracht werden.

Die Anwendung des erfindungsgemäßen Dentalwerkstoffs erfolgt beispielsweise wie folgt: Die Zahnhartsubstanz wird nach der Präparation abgespült und leicht trocken geblasen. Der erfindungsgemäße Dentalwerkstoff wird unmittelbar nach Vermischen der einzelnen Pasten auf die zu klebende Fläche der Restauration gegeben und die Restauration auf den Zahn platziert. Gegebenenfalls werden auftretende Überschüsse durch Belichtung mit einer Polymerisationslampe polymerisiert und entfernt. Im Falle einer lichtundurchlässigen Restauration wird diese auf dem Zahn festgedrückt bis die Aushärtung erfolgt (z.B. mindestens 1, 2 oder 3 Minuten lang). Bei lichtdurchlässigen Restaurationen kann diese durch die Restauration hindurch mit einer Polymerisationslampe belichtet und damit der Dentalwerkstoff lichtgehärtet werden, z.B. mit Belichtungszeiten von 10 bis 30 s pro Seite, vorzugsweise von etwa 20 s pro Seite.

Es wurde überraschend festgestellt, dass durch die erfindungsgemäße Aufteilung der basischen und nichtbasischen Füllstoffe auf die einzelnen Pasten des Mehrkomponentensystems eine deutliche Verbesserung der Dentinhaftung des ausgehärteten Dentalwerkstoff gegenüber bekannten selbsthaftenden Paste/Paste-Systemen erreicht wird. Der erfindungsgemäße selbsthaftende Dentalwerkstoff weist typischerweise eine Dentinhaftung nach Selbsthärtung bei Raumtemperatur von mindestens 4 MPa, vorzugsweise mindestens 5 MPa und besonders bevorzugt mindestens 6,5 MPa und nach Photohärtung von mindestens 7 MPa, vorzugsweise mindestens 8 MPa und besonders bevorzugt mindestens 9,5 MPa auf.

Überraschenderweise wurde gefunden, dass für ein funktionierendes selbsthaftendes Komposit auf Paste/Paste-Basis die Wechselwirkung zwischen Füllstoff und saurer Matrix eine entscheidende Rolle spielt. Scheinbar ist in Kompositen des Standes der Technik, in denen basische Füllstoffe in einer sauren Monomermatrix vorliegen, die Wechselwirkung der sauren Adhäsivmonomere mit dem basischen Füllstoff ausreichend stark, um eine zumindest teilweise Deaktivierung der sauren Monomere zu bewirken, so dass diese die Zahnhartsubstanz nicht mehr im notwendigen Maß anätzen können und die Dentinhaftung herabgesetzt wird. Erfindungsgemäße nichtbasische Füllstoffe sind daher Füllstoffe, die keine oder nur sehr geringe Wechselwirkungen mit den sauren Monomeren eingehen. Bei der vorliegenden Erfindung tritt vorzugsweise keine Wechselwirkung der säuregruppenhaltigen radikalisch polymerisierbaren Monomere (iii) mit dem nichtbasischen Füllstoff (iv) auf. Da eine Wechselwirkung zwischen Füllstoff und sauren Monomeren vermieden wird, besitzt der erfindungsgemäße mehrkomponentige Dentalwerkstoff eine sehr gute Lagerstabilität, d.h. auch nach Lagerung zeigt er noch sehr gute Dentinhaftung.

Im Falle von phosphorhaltigen sauren Monomeren, d.h. solchen mit Phosphorsäure- oder Phosphonsäuregruppen, kann der Nachweis einer Wechselwirkung durch ³¹P-Spektroskopie erfolgen. Hierzu wird der frisch hergestellten Paste CDCl₃ zugesetzt (Mischungsverhältnis Paste : CDCl3 = 1 : 1), aufgerührt und zentrifugiert. Mit der überstehenden Lösung wird ein ³¹P-NMR Spektrum aufgenommen. Liegt keine Wechselwirkung zwischen saurer Matrix und Füllstoff vor, wird ein scharfes Signal bei ca - 1-2 ppm im Fall von Phosphorsäuremonoestern und -diestern und - 30 ppm im Fall von Organophosphonsäuren und Organophosphonsäuremonoester erhalten, das dem Signal der reinen phosphorhaltigen Verbindung entspricht. Wechselwirkungen zwischen Füllstoff und saurer Matrix resultieren in einer Peakverbreiterung gegenüber dem Signal der reinen phosphorhaltigen Verbindung. Jeder Füllstoff der keine Verbreiterung des Peaks bewirkt, ist auf ein geeigneter nichtbasischer Füllstoff (iv) im Sinne der vorliegenden Erfindung.

Die Erfindung wird im Folgenden anhand von Beispielen näher erläutert. Bei den Prozentangaben handelt es sich jeweils um Gewichtsprozent.

### Ausführungsbeispiele

Die Pasten der Beispiele 1 und 2 mit den unten angegebenen Zusammensetzungen wurden wie folgt hergestellt. Wenn nicht anders angegeben handelt es sich bei allen Angaben um Gewichtsangaben (Gewichtsanteile). Zuerst wurden die Matrixmischungen hergestellt, indem die einzelnen Monomere zusammengerührt wurden Initiator und gegebenenfalls Aktivator in der Monomermischung gelöst wurde. In einem Rührgerät wurden die Matrixmischungen vorgelegt und die Füllstoffe eingerührt, bis homogene Pasten vorlagen. Zur Bestimmung der Dentinhaftung wurden die hergestellten Pasten (A) und (B) der Beispiele 1 und 2 jeweils 1:1 gemischt (da die Dichte der beiden Pasten annähernd gleich war, entsprach hier 1:1-Volumenverhältnis etwa 1:1-Gewichtsverhältnis).

### Messung der Dentin- und Schmelzhaftung

Die Haftung an der Zahnhartsubstanz wurde mit der sogenannten Ultradent-Methode durchgeführt. Hierzu wurden Rinderzähne in Polyesterharz eingebettet. Je nachdem, ob die Haftung auf Schmelz oder Dentin untersucht werden sollte, wurde die oberste Schmelzfläche mit 120 grid Sandpapier unter Wasser angeschliffen und mit 600 grid Sandpapier unter Wasser nachgeschliffen. Im Falle der Dentinhaftung wurde mit 120 grid Sandpapier so lange geschliffen, bis die Dentinoberfläche frei vorliegt. Vor der Präparation des Prüfkörpers für die Haftungsuntersuchung wurde die Zahnoberfläche mit Wasser gespült und mit einem Luftbläser leicht angetrocknet, keineswegs trocken geblasen. Es wurde ein vorpolymerisierter Kompositblock aus Tetric EvoCeram (lichthärtendes Füllungsmaterial von Ivoclar Vivadent AG, Liechtenstein) mit einer Länge von 3 mm und einem Durchmesser von 2,3 mm durch Bestrahlung (20 Sekunden) mit einer dentalen Polymerisationslampe (Bluephase, Ivoclar Vivadent AG, 400 - 500 nm) hergestellt. Anschließend wurde der Formkörper an der Haftfläche mit wenig Befestigungskomposit bestrichen und auf der Dentinoberfläche platziert. Der Kompositprüfkörper wurde mittels einer Schraube auf der Zahnoberfläche fixiert und die Zementüberschüsse wurden entfernt.

Zur Bestimmung der Dentin- bzw. Schmelzhaftung nach Selbsthärtung wurde die Prüfkörperanordnung 15 Minuten bei 37 °C im Trockenschrank gelagert, zur Bestimmung der Haftung nach Lichthärtung wurde die Zementfuge nach dem Entfernen der Zementüberschüsse 20 Sekunden mit einer Polymerisationslampe (Bluephase, Ivoclar Vivadent AG) belichtet. Anschließend wurden die Körper ausgeformt (von der Schraubenfixierung befreit) und in Wasser bei 37°C gelagert. Nach 24 Stunden wurde der Kompositprüfkörper von der Zahnoberfläche abgeschert mit einer Vorschubgeschwindigkeit von 0,8 mm/min. Eine Messserie umfasste 8 Prüfkörper, wobei der Mittelwert und die Standardabweichung berechnet wurden.

### Beispiel 1

### Paste (A)

| **Anteil** | **Komponente** |
|---|---|
| 25,0 % | Matrixmischung der Paste (A) |
| 71,4 % | Barium-Alumium-Fluoro-Silicat-Glasfüller G018-056*) 7 µm (Schott AG, Mainz; gewichtsmittlerer Partikeldurchmesser 7 µm); ca. 5 % Silan**) |
| 2,1 % | Barium-Alumium-Fluoro-Silicat-Glasfüller G018-056*) 1 µm (Schott AG, Mainz; gewichtsmittlerer Partikeldurchmesser 1 µm); ca. 5 % silan**) |
| 1,5 % | pyrogene Kieselsäure HDK® H2000 (Wacker Chemie AG, München; hydrophobiert durch Oberflächenmodifizierung mit Trimethylsiloxy, BET-Oberfläche von 170 - 230 m²/g) |

| | |
|---|---|
| *) 24 % SiO₂, 23 % Al₂O₃, 2 % Na₂O, 15 % CaO, 8 % P₂O₅, 11 % BaO, 17 % F **) 94 % Füller, 5 % Silan und 1 % Wasser werden gemischt und nach der Reaktion nicht reagiertes Silan verdunstet | |

### Matrixmischung der Paste (A) :

| **Anteil** | **Komponente** |
|---|---|
| 34,0 % | TEGDMA (Triethylenglykoldimethacrylat) |
| 10,0 % | PEG-400-Dimethacrylat |
| 51,6 % | UDMA (1,6-Bis[2-methacryloyloxyethoxycarbonylamino]-2,4,4-trimethylhexan) |
| 0,3 % | Campherchinon |
| 0,6 % | 4-(Dimethylamino)-benzoesäureethylester |
| 3,5 % | N,N-Dimethyl-sym,-xylidin |

### Paste (B)

| **Anteil** | **Komponente** |
|---|---|
| 35,0 % | Matrixmischung der Paste (B) |
| 24,5 % | Isofüller aus Monomer, pyrogener Kieselsäure und Ytterbiumfluorid* |
| 39,0 % | Ytterbiumfluorid (Partikelgröße 200 nm) |
| 1,5 % | pyrogene Kieselsäure HDK® H2000 |

| | |
|---|---|
| * Der Isofüller wurde hergestellt aus einem Komposit bestehend aus: | |

| | |
|---|---|
| 30 % | Matrixmischung aus |
| 78,5 % | UDMA |
| 21,0 % | Decandioldimethacrylat |
| 0,5 % | Benzolyperoxid |
| 50 % | pyrogener Kieselsäure Aerosil® OX-50 (Evonik Degussa; Partikelgröße 40 nm) |
| 10 % | Ytterbiumtrifluord, |

indem das Komposit bei 100 °C ca. 2 Stunden in Fladen (Dicke 1 cm) polymerisiert und anschließend gemahlen wurde (mittlere Partikelgröße nach dem Mahlen 5 µm)

### Matrixmischung der Paste (B) :

| **Anteil** | **Komponente** |
|---|---|
| 10,0 % | MDP (10-Methacryloyloxydecyldihydrogenphosphat) |
| 30,0 % | TEGDMA |
| 8,0% | PEG 400 Dimethacrylat |
| 50,5 % | UDMA |
| 1,5 % | Benzoylperoxid |

### Dentinhaftung:

| | |
|---|---|
| Nach Selbsthärtung: | 8,2 ± 1,8 MPa |
| Nach Lichthärtung: | 12,0 ± 2,0 MPa |

### Beispiel 2:

### Paste (A)

| **Anteil** | **Komponente** |
|---|---|
| 25,0 % | Matrixmischung der Paste (A) |
| 71,4 % | Barium-Alumium-Fluoro-Silicat-Glasfüller G018-056 7µm (wie Bsp. 1) |
| 2,1 % | Barium-Alumium-Fluoro-Silicat-Glasfüller G018-056 1µm (wie Bsp. 1) |
| 1,5 % | pyrogene Kieselsäure HDK® H2000 |

### Matrixmischung der Paste (A) :

| **Anteil** | **Komponente** |
|---|---|
| 34,0 % | TEGDMA |
| 10,0 % | PEG 400 Dimethacrylat |
| 51,6 % | UDMA |
| 0,3 % | Campherchinon |
| 0,6 % | 4-(Dimethylamino)-benzoesäureethylester |
| 3,5 % | N,N-Dimethyl-sym,-xylidin |

### Paste (B)

| **Anteil** | **Komponente** |
|---|---|
| 40,4 % | Matrixmischung der Paste (B) |
| 45,3 % | Sol-Gel-Silica ("Sunspheres" von Asahi Glass Co., Ltd, mittlere Partikelgröße 200 nm) |
| 12,6 % | Ytterbiumfluorid |
| 1,7 % | pyrogene Kieselsäure HDK® H2000 |

### Matrixmischung der Paste (B) :

| **Anteil** | **Komponente** |
|---|---|
| 10, 0 % | MDP |
| 22,8 % | TEGDMA |
| 7,6 % | PEG 400 Dimethacrylat |
| 33,7 % | Propoxyliertes Bisphenol-A Dimethacrylat |
| 24,4 % | UDMA |
| 1,5 % | Benzoylperoxid |

### Dentinhaftung:

| | | |
|---|---|---|
| Nach Selbsthärtung: | 7.2 ± 2.0 | MPa |
| Nach Lichthärtung: | 10.2 ± 2.7 | MPa |

Die Pasten der Beispiele 1 und 2 zeigten selbst nach Lagerung über einen Zeitraum von 2 Monaten noch sehr gute Dentinhaftwerte. Die nach Lagerung der Pasten aus Beispiel 2 bei Raumtemperatur bzw. bei 37°C erzielten Messwerte sind in den folgenden Tabellen zusammengestellt.

### Lagerung bei Raumtemperatur:

| **Lagerzeit** | **Selbsthärtung (MPa)** | **Lichthärtung (MPa)** |
|---|---|---|
| Initial | 7.7 ± 2.1 | 10.8 ± 2.9 |
| 1 Monat | 10.1 ± 1.6 | 10.3 ± 2.0 |
| 2 Monate | 9.2 ± 1.1 | 11.3 ± 2.3 |
| 3 Monate | 9.3 ± 1.4 | 11.2 ± 3.5 |

Die Daten bei Raumtemperatur sind konstant und zeigen keine Beeinträchtigung.

### Lagerung bei 37 °C:

| **Lagerzeit** | **Selbsthärtung (MPa)** | **Lichthärtung (MPa)** |
|---|---|---|
| Initial | 7.7 ± 2.1 | 10.8 ± 2.9 |
| 4 Wochen | 8.3 ± 2.0 | 11.2 ± 1.9 |
| 6 Wochen | 9.6 ± 1.3 | 9.1 ± 3.3 |

### Beispiel 3 (Vergleichsbeispiel)

Es wurde die Dentinhaftung des Befestigungskomposits Maxcem Elite™ der Kerr Corporation, USA, wie oben beschreiben untersucht. Soweit den Erfindern der vorliegenden Anmeldung bekannt, hat Maxcem Elite™ die folgende Zusammensetzung:

### Paste 1

### Füllstoffgehalt 77 %

### Füllerzusammensetzung:

| **Füllstoff** | **Gewichtsanteil** |
|---|---|
| Al₂O₃ | 9,6 % |
| SiO₂ | 59,2 % |
| BaO | 31,2 % |

### Matrixmischung:

| **Komponente** | **Gewichtsanteil** |
|---|---|
| Glycerinphosphorsäuredimethacrylat | 69 % |
| Pentaaerithritoltetraacrylat | 9,7% |
| Bis-GMA | 16,4 % |
| Unidentifizierbare Anteile | 4,9 % |

### Paste 2

### Füllstoffgehalt 77 %

### Füllerzusammensetzung:

| **Füllstoff** | **Gewichtsanteil** |
|---|---|
| F | 14,9 % |
| Al₂O₃ | 19,0 % |
| SiO₂ | 26,7 % |
| ZnO | 11,7 % |
| SrO | 22,8 % |
| ZrO₂ | 4,9 % |

### Matrixmischung:

| **Komponente** | **Gewichtsanteil** |
|---|---|
| UDMA | 73,6 % |
| Glycerindimethacrylat | 23,8% |
| 4-(Dimethylamino)-benzoesäureethyl ester | 1 % |
| Unidentifizierbare Anteile | 1,6 % |

### Dentinhaftung:

| | |
|---|---|
| Nach Selbsthärtung: | 2 - 4 MPa |
| Nach Lichthärtung: | 1 - 6 MPa |

Die deutlich schlechteren Dentinhaftwerte des Vergleichsbeispiels sind darauf zurückzuführen, das hier die Paste 1 im Gegensatz zur erfindungsgemäßen Lehre neben dem sauren Monomer Glycerinphosphorsäuredimethacrylat gleichzeitig den basischen Füllstoff Barium-Aluminium-Silicat-Glas enthält.

## Patentansprüche

1. Härtbarer mehrkomponentiger Dentalwerkstoff, umfassend
(A) eine erste Paste, enthaltend
(i) ein oder mehrere säuregruppenfreie radikalisch polymerisierbare Monomere,
(ii) basischen Füllstoff,
(vii) basischen Aktivator,
(viii)Photoinitiator und
(ix) nicht basischen Füllstoff,
(B) eine zweite Paste, enthaltend
(iii) ein oder mehrere säuregruppenhaltige radikalisch polymerisierbare Monomere,
(iv) nichtbasischen Füllstoff,
(v) Peroxid und
(vi) ein oder mehrere säuregruppenfreie radikalisch polymerisierbare Monomere,
wobei Paste (A) keine säuregruppenhaltigen radikalisch polymerisierbare Monomere und Paste (B) keinen basischen Füllstoff enthält und
wobei der Dentalwerkstoff kein zugesetztes Lösungsmittel enthält.

2. Dentalwerkstoff nach Anspruch 1, bei dem der nichtbasische Füllstoff (iv) und ggf. auch der nichtbasische Füllstoff (ix) ausgewählt ist aus SiO₂, neutralen Metallsalzen und deren Kombinationen.

3. Dentalwerkstoff nach Anspruch 2, bei dem der nichtbasische Füllstoff (iv) und/oder (ix) ausgewählt ist aus Quarz, pyrogener Kieselsäure, Fällungskieselsäure, Sol-Gel-Silica, Quarzglas, neutralen Fluoriden der Seltenerdmetalle und deren Kombinationen.

4. Dentalwerkstoff nach einem der vorstehenden Ansprüche, bei dem das mindestens eine säuregruppenhaltige radikalisch polymerisierbare Monomer (iii) mindestens eine ethylenisch ungesättigte Gruppe, ausgewählt aus Acryloyl-, Methacryloyl-, Vinylgruppen und deren Kombinationen, und mindestens eine Säuregruppe, ausgewählt aus Carbonsäure-, Sulfonsäure-, Phosphonsäure-, Phosphorsäuregruppen und deren Kombinationen, enthält.

5. Dentalwerkstoff nach Anspruch 4, bei dem das mindestens eine säuregruppenhaltige radikalisch polymerisierbare Monomer (iii) ein radikalisch polymerisierbares Dihydrogen- oder Hydrogenphosphat ist.

6. Dentalwerkstoff nach einem der vorstehenden Ansprüche, der mindestens ein säuregruppenfreies radikalisch polymerisierbares Monomer (i) und/oder (vi) enthält, das mindestens zwei ethylenisch ungesättigte Gruppen aufweist, die aus Acryloyl- und/oder Methyacryloylgruppen ausgewählt sind.

7. Verfahren zur Bereitstellung eines gebrauchsfertigen Dentalwerkstoffs durch Vermischen der Paste (A) und der Paste (B) des härtbaren mehrkomponentigen Dentalwerkstoffs nach einem der vorstehenden Ansprüche in einem Volumenverhältnis im Bereich von 0,4:0,6 bis 0,6:0,4.

8. Härtbarer mehrkomponentiger Dentalwerkstoff gemäß einem der Ansprüche 1 bis 6 zur Verwendung in der Zahnheilkunde.

9. Härtbarer mehrkomponentiger Dentalwerkstoff gemäß einem der Ansprüche 1 bis 6 zur Befestigung einer dentalen Restauration an Zahnschmelz und/oder Dentin.

10. Doppelspritze mit einer ersten und zweiten Vorratskammer, **dadurch gekennzeichnet, dass** sie einen härtbaren mehrkomponentigen Dentalwerkstoff gemäß einem der Ansprüche 1 bis 6 enthält, wobei Paste (A) in der ersten Vorratskammer und Paste (B) in der zweiten Vorratskammer enthalten ist.

## Claims

1. Curable multicomponent dental material, comprising
(A) a first paste, comprising
(i) one or more acid group-free radically polymerizable monomers,
(ii) basic filler,
(vii) basic activator,
(viii) photoinitiator and
(ix) non-basic filler,
(B) a second paste, comprising
(iii) one or more acid group-containing radically polymerizable monomers,
(iv) non-basic filler,
(v) peroxide and
(vi) one or more acid group-free radically polymerizable monomers,
wherein paste (A) does not comprise acid group-containing radically polymerizable monomers and paste (B) does not comprise basic filler and
wherein the dental material does not comprise added solvent.

2. Dental material according to claim 1, wherein the non-basic filler (iv) and optionally also the non-basic filler (ix) is selected from SiO₂, neutral metal salts and their combinations.

3. Dental material according to claim 2, wherein the non-basic filler (iv) and/or (ix) is selected from quartz, pyrogenic silica, precipitated silica, sol-gel silica, quartz glass, neutral fluorides of the rare-earth metals and their combinations.

4. Dental material according to one of the previous claims, wherein the at least one acid group-containing radically polymerizable monomer (iii) comprises at least one ethylenically unsaturated group, selected from acryloyl groups, methacryloyl groups, vinyl groups and their combinations, and at least one acid group, selected from carboxylic acid groups, sulfonic acid groups, phosphonic acid groups, phosphoric acid groups and their combinations.

5. Dental material according to claim 4, wherein the at least one acid group-containing radically polymerizable monomer (iii) is a radically polymerizable dihydrogen phosphate or hydrogen phosphate.

6. Dental material according to one of the previous claims comprising at least one acid group-free radically polymerizable monomer (i) and/or (vi), which possesses at least two ethylenically unsaturated groups that are selected from acryloyl groups and/or methacryloyl groups.

7. Process for the preparation of a ready-for-use dental material by mixing the paste (A) and the paste (B) of the curable multicomponent dental material according to one of the previous claims in a volume ratio in the range 0.4: 0.6 to 0.6: 0.4.

8. Curable multicomponent dental material according to one of the claims 1 to 6 for use in dentistry.

9. Curable multicomponent dental material according to one of claims 1 to 6 for fixing a dental restoration to tooth enamel and/or dentine.

10. Double syringe with a first and second reservoir, **characterised in that** it comprises a curable multicomponent dental material according to one of the claims 1 to 6, wherein paste (A) is contained in the first reservoir and paste (B) in the second reservoir.

## Revendications

1. Matériau dentaire multicomposant durcissable, comprenant :
A) une première pâte qui contient
i) un ou plusieurs monomère(s) polymérisable(s) par voie radicalaire, ne comportant aucun groupe acide,
ii) une charge basique,
vii) un activateur basique,
viii) un photoamorceur,
ix) et une charge non-basique,
B) et une deuxième pâte qui contient
iii) un ou plusieurs monomère(s) polymérisable(s) par voie radicalaire, porteur(s) de groupe(s) acide(s),
iv) une charge non-basique,
v) un peroxyde,
vi) et un ou plusieurs monomère(s) polymérisable(s) par voie radicalaire, ne comportant aucun groupe acide,
dans lequel la pâte (A) ne contient aucun monomère polymérisable par voie radicalaire et porteur de groupe(s) acide(s) et la pâte (B) ne contient pas de charge basique,
et lequel matériau dentaire ne contient aucun solvant ajouté.

2. Matériau dentaire conforme à la revendication 1, dans lequel la charge non-basique (iv), ainsi que, le cas échéant, la charge non-basique (ix), est choisie parmi de la silice SiO₂, des sels métalliques neutres, et leurs combinaisons.

3. Matériau dentaire conforme à la revendication 2, dans lequel la ou les charges non-basiques (iv) et/ou (ix) est ou sont choisie(s) parmi les suivantes : quartz, silice de pyrohydrolyse, silice précipitée, silice sol-gel, verre de quartz, et fluorures neutres des métaux des terres rares, ainsi que leurs combinaisons.

4. Matériau dentaire conforme à l'une des revendications précédentes, dans lequel le monomère (iii) polymérisable par voie radicalaire au nombre d'au moins un, porteur de groupe(s) acide(s), comporte au moins un groupe à insaturation éthylénique, choisi parmi les groupes acryloyle, méthacryloyle et vinyle et leurs combinaisons, et au moins un groupe acide choisi parmi les groupes acide carboxylique, acide sulfonique, acide phosphonique et acide phosphorique et leurs combinaisons.

5. Matériau dentaire conforme à la revendication 4, dans lequel le monomère (iii) polymérisable par voie radicalaire au nombre d'au moins un, porteur de groupe(s) acide(s), est un dihydrogénophosphate ou hydrogénophosphate polymérisable par voie radicalaire.

6. Matériau dentaire conforme à l'une des revendications précédentes, qui contient au moins un monomère (i) et/ou (vi) polymérisable par voie radicalaire et ne comportant aucun groupe acide, qui comporte au moins deux groupes à insaturation éthylénique, choisis parmi les groupes acryloyle et/ou méthacryloyle.

7. Procédé de préparation d'un matériau dentaire prêt à servir, dans lequel on mélange, en un rapport en volume de 0,4/0,6 à 0,6/0,4, de la pâte (A) et de la pâte (B) d'un matériau dentaire multicomposant durcissable conforme à l'une des revendications précédentes.

8. Matériau dentaire multicomposant durcissable, conforme à l'une des revendications 1 à 6, pour utilisation en chirurgie dentaire.

9. Matériau dentaire multicomposant durcissable, conforme à l'une des revendications 1 à 6, pour fixation d'une restauration dentaire sur l'émail dentaire et/ou la dentine.

10. Canule double, dotée d'un premier compartiment de réserve et d'un deuxième, **caractérisée en ce qu'**elle contient un matériau dentaire multicomposant durcissable, conforme à l'une des revendications 1 à 6, étant entendu que la pâte (A) est contenue dans le premier compartiment de réserve, et la pâte (B), dans le deuxième compartiment de réserve.
